# FASCICULE DE BREVET EUROPEEN

(11) **EP 0 725 627 B1**
(45) Date de publication et mention de la délivrance du brevet: **23.06.1999**
(21) Numéro de dépôt: 95900166.0
(22) Date de dépôt: 27.10.1994
(51) Int. Cl.: A61K 9/00

(54) **FORME GALENIQUE A ADMINISTRATION ORALE POUR ANIMAUX, SON PROCEDE DE PREPARATION ET SES APPLICATIONS**
ORALE DOSIERUNGSFORM FÜR TIERE, VERFAHREN ZU IHRER HERSTELLUNG UND VERWENDUNGEN
ORALLY-ADMINISTERED DOSAGE FORM FOR ANIMALS, PREPARATION METHOD THEREFOR AND USES THEREOF

(30) Priorité: 29.10.1993 FR 9312954
(43) Date de publication de la demande: 14.08.1996
(73) Titulaire: VIRBAC, 06516 Carros (FR)
(72) Inventeur: DERRIEU, Guy, F-06800 Cagnes-sur-mer (FR); AUBERT, André, F-06570 Opio (FR); RAYNIER, Bernard, F-06200 Nice (FR); SCHUMACHER, Carolin L., F-06140 Vence (FR)
(74) Mandataire: Ores, Irène
(86) Numéro de dépôt international: FR9401251
(87) Numéro de publication internationale: WO9511665

(56) Documents cités:
- EP-A- 0 458 751
- EP-A- 0 475 536

## Description

La présente invention est relative à une forme galénique destinée à permettre l'administration orale de substances chimiques ou médicamenteuses, telles que vitamines, oligo-éléments, amino-acides, substances nutritionnelles, vaccins etc..., à des animaux domestiques ou sauvages.

La présente invention est également relative au procédé d'obtention desdites formes galéniques.

On connaît déjà des systèmes permettant l'administration orale de médicaments à des animaux domestiques ou à des animaux élevés de manière extensive, ou à des animaux sauvages, difficiles ou dangereux à contenir.

De tels systèmes ont fait l'objet de Demandes de Brevet ou de Brevets (Brevet EP 0 240 826, Brevet EP 0 208 528 et Demande de Brevet EP 0 421 863).

L'appât décrit dans le Brevet EP 0 240 826 est obtenu par une première coulée, au fond d'un moule, d'un support comprenant un composé lipidique (ayant un point de fusion entre 20° et 60°C), un composé destiné à stabiliser la forme de l'appât et un composé attractif et appétant pour les animaux, une mise en place de la substance active sur la couche de support solidifiée, puis une deuxième coulée dudit support, de manière à recouvrir complètement ladite substance active.

Ce système a l'inconvénient de présenter une faible résistance mécanique et une fragilité au voisinage de la jonction des deux coulées et le rend inapte aux modes de distribution à grande échelle (largage aérien, par exemple, pour traiter des effectifs importants d'animaux sauvages, sur des territoires importants).

Le Brevet EP 208 528 décrit un appât pour poissons et crustacés constitué essentiellement d'un polymère insoluble dans l'eau ayant un point de fusion inférieur à 110°C (polyamides ou copolymères d'éthylène, EVA, en particulier), d'une substance attractive et d'huile comestible ou de mélasse (0 à 20 %).

Les appâts selon ce Brevet EP 208 528 sont obtenus par extrusion à 90-110°C d'un mélange à sec du polymère, de la substance attractive et éventuellement de l'huile comestible.

La Demande Internationale WO 89/12393 décrit des compositions pesticides comprenant de l'EVA, un agent bioactif, une source de protéines/sucres/lipides et éventuellement 0 à 20 % d'huile comestible, une substance attractive, un colorant, un conservateur, un agent répulsif et un biomarqueur.

Les compositions selon cette Demande Internationale PCT WO 89/12393 peuvent être sous forme de blocs ou de comprimés et sont également préparées par extrusion de la composition précitée, soit par fusion du polymère (fusible à une température < 110°C), suivie du mélange de ce dernier avec les autres ingrédients, soit par chauffage, jusqu'au point de fusion du polymère, d'un mélange de l'ensemble des ingrédients sous forme sèche.

La Demande de Brevet EP 421 863 décrit des systèmes comprenant deux parties : une enveloppe sous forme tubulaire, obtenue par extrusion et comprenant au moins une substance attractive, au moins une substance agglomérante (polyosides, amidons ou polymères tels que EVA) et éventuellement une substance hydrophobe (huile) et à l'intérieur de la cavité de l'enveloppe, une substance de liaison (mélange de corps gras possédant un point de fusion peu élevé) contenant un principe actif, la substance de liaison épousant la forme interne de l'enveloppe. Une telle enveloppe possède une résistance mécanique et thermique élevée, permettant notamment la distribution par largage aérien.

Les compositions ou systèmes de l'Art antérieur sont généralement conçus de telle façon qu'ils présentent une bonne résistance à la manipulation et aux chocs et/ou une bonne attractivité vis-a-vis des animaux, facilitant la prise effective du (ou des) principe(s) actif(s) inclus dans ces systèmes.

Toutefois, de façon générale, les compositions décrites dans ces documents n'assurent pas aux principes actifs un temps de séjour dans la cavité buccale suffisant pour garantir l'efficacité de certains médicaments ou lorsqu'il est indispensable d'obtenir un certain temps de contact, notamment pour certains traitements. De plus, elles n'assurent pas aux principes actifs une stabilité satisfaisante, en particulier à ceux d'origine biologique.

La Demande EP 458 751 décrit un système comprenant un noyau central comprenant des aminoacides cycliques, un premier enrobage composé d'un polymère apte à former un film (5 %-100 % du noyau en poids) et un deuxième enrobage hydrophobe consistant en lipides (20 %-400 % en poids de la combinaison noyau+ premier enrobage) permettant l'obtention de granules, essentiellement destinés à être soumis à une autre transformation ; en outre, de tels systèmes induisent une libération contrôlée du principe actif et ne permettent pas de transformer et de stabiliser des produits d'origine biologique en solution ou suspension aqueuse.

La présente invention s'est, en conséquence, donné pour but de fournir une nouvelle forme galénique qui répond mieux aux exigences rencontrées dans le traitement de certaines affections, dans la dispensation de certains soins, dans l'exécution de certains actes médicaux sur les animaux, tels que par exemple la vaccination orale ou les traitements de la cavité buccale, que les compositions et systèmes de l'Art antérieur.

La présente invention a pour objet une forme galénique pour l'administration orale de substances chimiques ou médicamenteuses, du type comprenant un noyau central solide contenant une ou plusieurs substances bioactives et une couche externe ou enrobage, caractérisée en ce que :
- le noyau central, hydrosoluble, et poreux comprend :
   . au moins une substance choisie dans le groupe qui comprend les liants sélectionnés parmi des polypeptides, des polysaccharides à poids moléculaire élevé, des polymères pouvant donner des solutions colloïdales et des colloïdes, et les diluants sélectionnés parmi des polyols, des oxydes métalliques, des carbonates, des phosphates ou la cellulose microcristalline, la quantité globale de liant et de diluant étant comprise entre 50 et 98 % en poids par rapport audit noyau central et
   . une quantité efficace d'au moins une substance bioactive et
- la couche externe, à caractère hydrophobe et appétant, comprend :
   . au moins une substance lipidique choisie parmi les alcools gras (alcool cétylique, alcool stéarylique), les acides gras (acide stéarique, acide palmitique), les esters de glycérol tels que le palmitostéarate de glycérol, le stéarate de glycérol (commercialisé sous la marque PRECIROL), le béhénate de glycérol (commercialisé sous la marque COMPRITOL), les huiles hydrogénées telles que l'huile de ricin hydrogénée (commercialisée sous la marque CUTINA HR), les cires ou corps gras tels que cire de carnauba, cire d'abeille, paraffine, lanoline, huile de coprah et les sels d'acides gras tels que stéarate de calcium ou de magnésium ;
   . un agent de modulation du délitement de ladite couche externe et de l'adhésion des fragments microparticulaires issus de la désintégration du noyau central, soluble ou dispersible dans la masse lipidique, choisi parmi des polymères sélectionnés dans le groupe qui comprend des résines acryliques (commercialisées sous la marque EUDRAGIT), des acétates de polyvinyle (commercialisés sous la marque RHODOPAS), des résines de polyalkylène, des copolymères éthylène/acétate de vinyle (EVA), les polymères réticulés d'amidon, de dextran, d'inuline ou de vinylpyrrolidone et des dérivés cellulosiques sélectionnés parmi l'hydroxypropylcellulose, l'hydroxypropylméthylcellulose, l'éthylcellulose, la carboxyméthylcellulose ; et
   . des substances appétentes, naturelles ou synthétiques, d'origine purement végétale ou contenant des produits d'origine animale, choisies parmi les farines de viande ou de poisson, les arômes tels que arôme boeuf bouilli, arôme porc rôti, arôme poisson blanc, arôme arachide, arôme bacon, arôme foie.

Cette forme galénique est donc à double compartiment. Le premier compartiment, à coeur, est un noyau central, solide et poreux, ayant pour caractéristiques de se dissoudre ou de se désintégrer rapidement en milieu aqueux ou dans la salive. Les agrégats ou fragments microparticulaires générés par cette désintégration sont susceptibles d'adhérer aux tissus de la cavité buccale, en raison de la composition particulière de la forme selon l'invention.

Le deuxième compartiment est en fait une pellicule ou enveloppe de nature à la fois lipidique et polymérique, hydrophobe, appétente et d'épaisseur contrôlée qui assure :
- une manipulation aisée de la forme galénique selon l'invention, sans modifier l'intégrité du compartiment central, hydrosoluble et de structure poreuse,
- une protection du noyau central contre l'humidité,
- une protection contre la contamination de la personne chargée de dispenser le produit, en particulier pour la vaccination orale,
- une attractivité vis-à-vis de l'animal cible,
- une bonne résistance thermique à des températures comprises entre -30° C et +45° C, sans modification de la texture, ni suintement lipidique,
- une stabilité optimale du noyau central solide et sec et donc des molécules bioactives sensibles,
- une stabilité dans le temps, les caractéristiques physiques étant non altérables, lors du séjour dans le milieu de distribution, plus particulièrement pour les animaux d'élevage intensif, et
- une bioadhésion aux muqueuses de la cavité buccale et une mise à disposition immédiate des principes actifs aux travers de celles-ci, ce qui conduit à une biodisponibilité optimale, alors que les comprimés ou autres appâts selon l'Art antérieur, ont un contenu trop compact ou trop fluide pour permettre une persistance dans la cavité buccale.

On entend par principe actif toute matière biologiquement active et plus spécialement toute molécule pouvant présenter des difficultés de formulation liées à des problèmes de goût, de faible solubilité ou d'insolubilité, d'instabilité, ou à une biodisponibilité faible.

La forme galénique selon l'invention peut être utilisée pour l'administration de toutes sortes de substances, communément utilisées en médecine vétérinaire et plus particulièrement aux vaccins oraux, aux agents de nutrition, aux agents régulateurs des métabolismes, aux contraceptifs, aux extraits de plantes, aux adjuvants d'alimentation, aux agents d'hygiène et de diététique, aux agents cosmétiques. Cette nouvelle forme permet, en outre, une présentation solide de principes actifs liquides.

A titre d'exemple, parmi les substances actives administrables sous cette forme, peuvent être cités :
- les antibiotiques tels que betalactamines (amoxicilline, ampicilline, cefalexine), chloramphénicol, macrolides (josamycine, érythromycine, spiramycine, tylosine), tétracyclines,
- les antiinfectieux autres que antibiotiques tels que furanes, quinolones, sulfamides, sulfones, triméthoprime,
- les antiinflammatoires tels que corticoïdes, pyrazoles, salicylés, non stéroidiens,
- les antimycosiques tels que griséofulvine, kétonazole,
- les anthelminthiques tels que oxibendazole, pyrantel, mébendazole, oxfendazole, fenbendazole, netobimin,
- les agents de nutrition, tels que réhydratants oraux, acides aminés (méthionine, lysine) produits d'apports énergétiques minéraux ou vitaminiques,
- les produits locaux gingivodentaires, antiseptiques, antibactériens, tels que hexamidine, chlorhexidine, hexitidine, et les substances modifiant l'haleine,
- les vaccins anti-viraux, anti-bactériens, anti-parasitaires,
   sous forme :
   . Inactivés : antigène complet
   . Vivants atténués
   . Vivants mutés avirulents
   . Vivant issus de recombinaison génétique (vecteur viraux, bactériens, plasmidiques)
   . Sous unitaires : (glycoprotéine ; nucléoprotéine)
   . Recombinants purifiés : molécules antigéniques pures
   . Peptidiques
   . Composés d'acide nucléiques
   . Composés de molécules biologiques associées à diverses cytokines : IL2, IL6, IL12, interféron ; TNF ...
   . Nucléosides anti-sens
   . Anticorps monoclonaux anti-idiotypiques ou pas
   . Récepteurs cellulaires spécifiques
- les cytokines,
- les anti-cancéreux,
- les facteurs de croissances,
- les vaccins contraceptifs hormonaux ou pas,
- les insecticides biologiques,
- les adjuvants
   . non spécifiques de l'immunité
   . spécifiques de TH1 (immunité cellulaire)
   . spécifiques de TH2 (immunité humorale).
- les facteurs, support de transgénie,
- les facteurs d'attachement ou anti-attachement locaux (muqueuses, villosités intestinales ...).

On entend par liant une substance épaississante et structurante servant de support, soluble ou dispersible dans l'eau, permettant d'assurer la cohésion de la masse, inerte vis-à-vis du (des) principe(s) actif(s) et susceptible de favoriser l'adhésion des fragments microparticulaires du noyau central aux muqueuses de la cavité buccale, après la prise effective de ladite forme galénique par l'animal.

Ces liants sont notamment choisis parmi les polypeptides tels que la gélatine ou la gélatine partiellement hydrolysée, des polysaccharides à poids moléculaire élevé, des polymères pouvant donner des solutions colloïdales et des colloïdes, tels que les gommes naturelles ou synthétiques, (la gomme arabique, la gomme caraya, la gomme xanthane, la gomme guar, la gomme de caroube), les alginates, les dérivés cellulosiques tels que la carboxyméthyl cellulose sodique, l'hydroxypropylméthyl cellulose, les pectinates, les carrageenanes, les dextranes, les homo ou copolymères de l'acide acrylique, les dérivés hydrodispersibles de l'amidon, les silices colloïdales, l'alcool polyvinylique, la polyvinylpyrrolidone, les polyéthylèneglycols (PEG 6000 et 8000 particulièrement), ou encore des mélanges desdits liants.

De manière avantageuse, ces liants sont plus particulièrement choisis parmi la gomme arabique, la gomme xanthane, la pectine, biopolymères naturels communément utilisés dans l'élaboration des comprimés ou gommes à mâcher pour améliorer la bioadhésion aux muqueuses de la cavité buccale.

On désigne par diluant des substances pharmaceutiquement acceptables, de préférence hydrosolubles, qui améliorent les propriétés physiques du noyau central. Ces substances peuvent être notamment choisies parmi le mannitol, le xylitol, le lactose, le glycocolle, le sorbitol, le glucose, les maltodextrines, les cyclodextrines, ou encore parmi les oxydes (oxyde de magnésium), les carbonates (carbonate de calcium), les phosphates (phosphate tricalcique), la cellulose microcristalline.

Selon un mode de réalisation avantageux de ladite forme galénique, le noyau central comprend en outre un ou plusieurs additifs choisis parmi des substances modifiant ou masquant le goût telles que la saccharine, les saccharinates, les cyclamates, l'aspartame, des agents modulant le délitement comme la silice, des promoteurs d'absorption tels que les cyclodextrines ou les sels de l'acide glycyrrhétinique, des agents de surface choisis parmi des tensio-actifs non ioniques ou cationiques, tels que les esters de sorbitane, les copolymères d'oxyde d'éthylène et de propylène, les éthers de polyoxyéthylène et d'alcool gras, des agents colorants et des agents conservateurs.

Le noyau central contient donc au moins une substance choisie parmi les liants et diluants énumérés ci-dessus, mais il peut contenir un ou plusieurs liants et/ou un ou plusieurs diluants.

Selon un autre mode de réalisation avantageux de ladite forme galénique, la substance lipidique de ladite couche externe est choisie parmi la paraffine, l'huile de coprah, l'acide palmitique et les esters du glycérol et l'agent de modulation du délitement de ladite couche externe est choisi parmi les copolymères éthylène/acétate de vinyle et les polysaccharides réticulés.

Conformément à l'invention, la couche externe ou barrière hydrophobe de la forme galénique selon l'invention pourra également contenir :
- des substances minérales choisies dans le groupe qui comprend les oxydes minéraux sélectionnés parmi l'oxyde de titane et de fer, les phosphates, les carbonates, les argiles et le talc, lesquelles substances agissent également sur la vitesse de délitement de ladite couche externe et/ou
- un agent tensio-actif choisi par exemple parmi les esters de sorbitol, les polysorbates de polyoxyéthylène (commercialisés sous la marque TWEEN), les lécithines, les esters de sorbitane, pour garantir l'homogénéité et la transformation du mélange la constituant, lorsque celle-ci se présente sous la forme d'une suspension lors de l'opération de pelliculage (d'enrobage) du noyau central. Cette matière hydrophobe pourra avantageusement contenir des esters du glycérol et d'acides gras, tels que mono et (ou) diglycérides, dont l'incidence majeure sera d'accroître l'affinité entre la couche lipidique et le noyau central, favorisant ainsi l'adhésion entre les deux compartiments de la forme galénique selon l'invention. La flexibilité et l'élasticité relative de la matière filmogène s'en trouvent renforcées.

Conformément à l'invention, ladite forme galénique comprend avantageusement :

| - noyau central : | |
|---|---|
| diluants et/ou liants | 50 à 98 % |
| substances bioactives | 0,25 à 50 % |
| autres additifs | 0 à 1,75 % |

| - couche externe : | |
|---|---|
| substances lipidiques | 40 à 93 % |
| polymères | 4 à 30 % |
| substances appétentes et autres additifs | 3 à 30 % |

La présente invention a également pour objet un procédé de préparation de la forme galénique conforme à l'invention, caractérisé en ce que l'on effectue les opérations suivantes :
a) préparation d'une pâte contenant les différents constituants du noyau central, à savoir, substances bioactives, diluants, liants et éventuellement un ou plusieurs additifs et une quantité d'eau convenable pour régler l'homogénéité et la viscosité de la suspension obtenue,
b) solidification du produit obtenu en a),
c) enrobage des produits obtenus en b), avec un mélange en solution ou en suspension, comprenant au moins une substance lipidique, un agent de modulation du délitement de la couche externe et une substance appétente.

De manière plus précise, la préparation du noyau central se déroule selon les opérations suivantes :
a) préparation d'une pâte contenant les différents constituants énumérés plus avant, ainsi qu'une quantité d'eau convenable de manière à assurer à la suspension obtenue des caractéristiques rhéologiques permettant une transformation optimale
b) solidification du produit obtenu en a), notamment par congélation et sublimation (lyophilisation) en particulier lorsque le principe actif est un vaccin, ou opération équivalente.

Selon un mode de mise en oeuvre avantageux dudit procédé, ladite solidification est réalisée par une opération physique telle ou'évaporation, dessiccation ou lyophilisation.

Lorsque la solidification est réalisée par évaporation, celle-ci est réalisée sous pression réduite et de préférence associée à une exposition aux hyperfréquences. Cette transformation, réalisée par exemple dans un mélangeur sécheur VRIECO-NAUTA ou VACTRON (MACHINES COLETTE), sera préférée lorsqu'on souhaitera obtenir une unité de forme sphérique ou globalement sphérique, les conditions d'utilisation permettant de moduler la taille moyenne du noyau central selon l'invention.

Lorsque la solidification est réalisée par lyophilisation, la pâte obtenue en a) est de préférence divisée en quantités unitaires de forme et de volume déterminés avant congélation et sublimation.

Il est entendu que la division du produit peut également être effectuée mécaniquement après lyophilisation, mais il est avantageux de répartir la pâte dans des alvéoles de forme et de dimensions prédéterminées, préalablement à l'opération de lyophilisation.

Conformément à l'invention, le(s) principe(s) actif(s) seront solubles ou en suspension dans la pâte à répartir, sous forme libre ou sous forme de microparticules ou nanoparticules. Cette dernière forme, plus particulièrement adaptée à une solidification par lyophilisation, peut éviter le relargage complet et immédiat du principe actif lors de la mise en contact avec les milieux aqueux et le flux salivaire. Des polymères biorésorbables ou des substances macromoléculaires entrent dans la constitution des micro ou nanoparticules.

L'enrobage du noyau central précédemment décrit est effectuée selon les techniques usuelles communément employées dans l'industrie pharmaceutique et alimentaire. Selon la forme et la dimension du noyau central, on utilisera plus particulièrement l'enrobage par évaporation du solvant, l'enrobage par coacervation, l'enrobage par pelliculage en turbine, l'enrobage en lit d'air fluidisé ou l'enrobage par trempage largement employé dans l'industrie alimentaire, plus précisément la confiserie.

La forme galénique conforme à l'invention est tout particulièrement indiquée :
- pour les formulations pharmaceutiques orales dont il convient de masquer le goût des substances bioactives,
- pour les formulations à bio-adhésion buccale, comme par exemple les systèmes de rafraîchissement de l'haleine,
- pour les formulations incluant des principes actifs sensibles aux mécanismes de la digestion et dont on stimulera le passage par les muqueuses de la cavité buccale.

Outre les dispositions qui précèdent, l'invention comprend encore d'autres dispositions, qui ressortiront de la description qui va suivre, qui se réfère à des exemples de mise en oeuvre du procédé objet de la présente invention.

Il doit être bien entendu, toutefois, que ces exemples sont donnés uniquement à titre d'illustration de l'objet de l'invention, dont ils ne constituent en aucune manière une limitation.

### EXEMPLE I : Forme unitaire pour l'hygiène dentaire.

Dans un mélangeur planétaire de type OLSA, on prépare un pré-mélange contenant les principes actifs et excipients et ayant la composition suivante :

| | |
|---|---|
| Chlorhexidine diacétate | 0,25 % |
| Méthionine | 3 % |
| Lactose | 80 % |
| β-cyclodextrine | 13 % |
| Dextran 70 | 2 % |
| Silice pulvérisée | 0,25 % |
| Aspartame | 0,8 % |
| Monopalmitate de sorbitane | 0,7 % |

La masse pulvérulente est mélangée à sec pendant 30 minutes puis additionnée d'eau, à raison de 38 g pour 100 g de masse sèche, et malaxée pendant 1 heure à température ambiante. La suspension homogène ainsi obtenue est divisée dans des alvéoles de chlorure de polyvinyle de 1,5 cm3. La feuille alvéolée contenant la suspension est introduite dans un lyophilisateur immédiatement après division, pour être congelée à une température de -45° C sous pression atmosphérique, pendant environ 3 heures. Après dessiccation pendant 12 heures sous pression réduite à une température négative, la température est remontée à 30° C par paliers de 5° C. L'unité lyophilisée, extraite de l'alvéole, est traitée en lit d'air fluidisé (GLATT GPC-15) avec injection d'air (100 litres/min, à 30-5° C), par une solution d'huile de castor hydrogénée (1 partie), d'éthyl cellulose (2,5 parties), de cire d'abeille (3 parties), d'alcool cétylique (1 partie), d'arôme arachide (0,15 partie) dans le chlorure de méthylène (80 parties), maintenu à 25-30°C.

### EXEMPLE II : Forme unitaire pour la vaccination orale contre la rage chez le chien.

En opérant comme décrit précédemment pour l'exemple I, on engage un mélange sec ayant la composition suivante :

| | |
|---|---|
| Lactose | 28 % |
| Mannitol | 70 % |
| Gomme arabique | 2 % |

On additionne une suspension vaccinale de souche SAG-2 de titre suffisant pour garantir l'immunité à l'animal vacciné, à raison de 40 g pour 100 g de masse sèche et on opère comme décrit plus haut. L'unité lyophilisée, extraite de l'alvéole, est enrobée par trempage, dans un mélange homogène maintenu à 56-60° C, de composition suivante : Paraffine 50-2° C (52 %), copolymère éthylène acétate de vinyle à 28 % d'acétate de vinyle (6 %), farine de viande (23 %), suif de boeuf (17 %), arôme bacon de ROBERTET (2 %). On obtient ainsi une forme galénique selon l'invention propre à la vaccination orale.

### EXEMPLE III :

Dans un mélangeur sécheur planétaire pouvant supporter une pression réduite et équipé d'un générateur d'hyperfréquences, on réalise un mélange ayant la composition suivante :

| | |
|---|---|
| Lactose | 20 % |
| Mannitol | 45 % |
| Polyvinyl pyrrolidone K 30 | 6 % |
| Phosphate bicalcique | 5 % |
| Sulfaméthoxypyridazine | 20 % |
| Triméthoprime | 4 % |
| Eau | 45 g pour 100 g de masse sèche |

On mélange pendant 1 heure, puis on effectue l'élimination de l'eau sous vide à l'aide de micro-ondes (excitation en discontinu avec des générateurs de puissance variable entre 1 et 4 KW), en ne dépassant pas 40°C. Les granules obtenus sont enrobés en lit d'air fluidisé par une pulvérisation d'une solution à 5 % d'Eudragit® L 100, contenant une faible quantité de monoglyceride d'acide gras et d'anhydride silicique anhydre, dans un mélange 1/1 de chlorure de méthylène et d'isopropanol, à raison de 30 ml/min.

### EXEMPLE IV : Tests in vitro et in vivo des formes galéniques conformes à l'invention obtenues selon les exemples I et II.

a) Tenue à la température
Les essais ont montré qu'à des températures comprises entre -30 C et +45°C, il n'y avait aucune modification de l'aspect ou de la structure. La forme galénique, placée sur un papier absorbant conservé à 45°C, se ramollit très légèrement, sans laisser apparaître aucun suintement, ni tache de graisse sur le papier ; en outre, les caractéristiques physiques du noyau central lyophilisé ne sont modifiées.
b) Attractivité, appétence et bioadhésion
Les essais d'attractivité ont été réalisés sur deux groupes de neuf chiens (trois petits, trois moyens, trois grands), en présentant respectivement la forme galénique selon l'exemple I (groupe A), le noyau central selon l'exemple I (groupe B), en ayant pris soin d'introduire de la Rhodamine B utilisée comme marqueur à raison de 0,2 % (p/p). Les résultats sont très favorables, avec une prise immédiate de 100 % et 80 % respectivement pour les groupes A et B. Il faut noter que, pour le groupe B, la prise est effective pour les 20 % restants dans les dix minutes qui suivent la présentation. Les essais d'appétence et de bioadhésion sont réalisés sur les groupes A et B et un groupe C comparable, auquel est présenté un comprimé classique de composition similaire à la forme galénique selon l'exemple I. Les résultats sont très favorables à la forme galénique selon l'invention, avec une intensité moyenne de la coloration de la cavité bucco-pharyngée (échelle croissante de 0 à 4) de 4, 4 et 2 respectivement pour les groupes A, B et C.
c) Stabilité
Les essais réalisés en b) ont été repris avec des formes identiques mais conservés six mois à 4°C. Les résultats sont tout à fait comparables.
Une étude de stabilité de la forme galénique selon l'exemple II a été conduite en suivant le titre de la suspension vaccinale, en fonction du temps et de la température de conservation. Cette étude a été menée en parallèle en comparaison avec un lot contrôle de suspension vaccinale, conservée sous forme liquide et conditionnée en blister. Les contrôles sont effectués dans les 7, 14 et 28 jours, 3 et 6 mois qui suivent la préparation de la suspension et de la forme galénique correspondante. On exprime la chute hebdomadaire de la dose infectieuse cyto-pathogène détruisant 50 % des cellules (DICP50), sur les deux préparations. Les résultats sont rassemblés dans le Tableau I et montrent l'intérêt particulier de la forme galénique par rapport à une présentation conventionnelle.

**TABLEAU I**

| | | | | | |
|---|---|---|---|---|---|
| Température de stockage composition | -80°C | -20°C | +4°C | +25°C | +35°C |
| SAG-2 liquide en blister | 0 | -0,025* | -0,19 | -0,76 | -1,82 |
| Forme galénique selon l'exemple II | 0 | 0 | -0,05 | -0,1 | -0,3 |

| | | | | | |
|---|---|---|---|---|---|
| * : chute de la DICP 50 exprimée en log/semaine | | | | | |

d) Efficacité
Une étude a été menée avec la forme galénique selon l'exemple II en comparaison avec un appât pour chien conventionnel présentant la charge vaccinale sous forme liquide en blister. Les 14 chiens retenus sont divisés en 4 groupes A, B, C et D. Le groupe A (4 chiens) reçoit la forme galénique selon l'invention, le groupe B (4 chiens) reçoit le noyau central lyophilisé (sans le compartiment protecteur), le groupe C (4 chiens) reçoit un appât classique, et le groupe D (2 chiens) n'est pas traité. Les chiens sont suivis pendant 30 jours, en contrôlant l'évolution du taux d'anticorps antirabiques. A 30 jours, les taux de séroconversion sont supérieurs à 0,5 UI pour 4, 3 et 3 chiens, respectivement dans les groupes A, B et C.
Ces résultats montrent la stabilité et l'efficacité de la forme galénique selon l'invention.

## Revendications

1. Forme galénique pour l'administration orale de substances chimiques ou médicamenteuses, du type comprenant un noyau central solide contenant une ou plusieurs substances bioactives et une couche externe ou enrobage, caractérisée en ce que :
- le noyau central, hydrosoluble, et poreux comprend :
. au moins une substance choisie dans le groupe qui comprend les liants sélectionnés parmi des polypeptides, des polysaccharides à poids moléculaire élevé, des polymères pouvant donner des solutions colloïdales et des colloïdes, et les diluants sélectionnés parmi des polyols, des oxydes métalliques, des carbonates, des phosphates ou la cellulose microcristalline, la quantité globale de liant et de diluant étant comprise entre 50 et 98 % en poids par rapport audit noyau central et
. une quantité efficace d'au moins une substance bioactive et
- la couche externe, à caractère hydrophobe et appétant, comprend :
. au moins une substance lipidique choisie parmi les alcools gras, les acides gras, les esters de glycérol, les huiles hydrogénées, les cires, la paraffine, la lanoline, l'huile de coprah et les sels d'acides gras ;
. un agent de modulation du délitement et de l'adhésion de ladite couche externe, soluble ou dispersible dans la masse lipidique, choisi parmi des polymères sélectionnés dans le groupe qui comprend des résines acryliques, des acétates de polyvinyle des résines de polyalkylène, des copolymères éthylène/acétate de vinyle (EVA), les polymères réticulés d'amidon, de dextran, d'inuline ou de vinylpyrrolidone et des dérivés cellulosiques sélectionnés parmi l'hydroxypropylcellulose, l'hydroxypropylméthylcellulose, l'éthylcellulose, la carboxyméthylcellulose ; et
. des substances appétentes, naturelles ou synthétiques.

2. Forme galénique selon la revendication 1, caractérisée en ce que le noyau central comprend en outre un ou plusieurs additifs choisis parmi des substances modifiant ou masquant le goût, des agents modulant le délitement, des promoteurs d'absorption, des agents de surface choisis parmi des tensio-actifs non ioniques ou cationiques, des agents colorants et des agents conservateurs.

3. Forme galénique selon la revendication 1 ou la revendication 2, caractérisée en ce que la couche externe comprend en outre des substances minérales choisies dans le groupe qui comprend des oxydes minéraux, des phosphates, des carbonates, les argiles et le talc et/ou un agent tensio-actif choisi parmi les esters de sorbitol, les polysorbates de polyoxyéthylène, les lécithines et les esters de sorbitane.

4. Forme galénique selon l'une quelconque des revendications 1 à 3, caractérisée en ce que les diluants et liants du noyau interne sont sélectionnés parmi la gomme arabique, la gomme xanthane, la pectine, les polyols du type sorbitol, xylitol, mannitol et leurs mélanges.

5. Forme galénique selon l'une quelconque des revendications 1 à 4, caractérisée en ce que la substance lipidique de ladite couche externe est choisie parmi la paraffine, l'huile de coprah, l'acide palmitique et les esters du glycérol et en ce que l'agent de modulation du délitement de ladite couche externe est choisi parmi les copolymères éthylène/acétate de vinyle et les polysaccharides réticulés.

6. Forme galénique selon l'une quelconque des revendications 1 à 5, caractérisée en ce que les substances bioactives du noyau interne sont choisies parmi les vaccins, les cytokines, les anti-cancéreux, les facteurs de croissance, les adjuvants de l'immunité, les facteurs support de transgénie, les facteurs d'attachement ou d'anti-attachement locaux.

7. Forme galénique selon la revendication 6, caractérisée en ce que le noyau interne comprend en tant que substance bioactive, un vaccin choisi parmi les vaccins anti-viraux, les vaccins anti-parasitaires, les vaccins anti-bactériens, les vaccins contraceptifs.

8. Forme galénique selon la revendication 6 ou la revendication 7, caractérisée en ce que le noyau interne comprend en tant que substance bioactive, une suspension vaccinale antirabique de souche SAG-2.

9. Forme galénique selon l'une quelconque des revendications 1 à 8, caractérisée en ce qu'elle présente la formule suivante :
| - noyau central : | |
|---|---|
| diluants et/ou liants | 50 à 98 % |
| substances bioactives | 0,25 à 50 % |
| autres additifs | 0 à 1,75 % |
| - couche externe : | |
|---|---|
| substances lipidiques | 40 à 93 % |
| polymères | 4 à 30 % |
| substances appétentes et autres additifs | 3 à 30 % |

10. Procédé de préparation d'une forme galénique selon l'une quelconque des revendications 1 à 9, caractérisé en ce que l'on effectue les opérations suivantes :
a) préparation d'une pâte contenant les différents constituants du noyau central, à savoir, substances bioactives, diluants, liants et éventuellement un ou plusieurs additifs et une quantité d'eau convenable pour régler l'homogénéité et la viscosité de la suspension obtenue,
b) solidification du produit obtenu en a),
c) enrobage des produits obtenus en b), avec un mélange en solution ou en suspension, comprenant au moins une substance lipidique, un agent de modulation du délitement de la couche externe et une substance appétente.

11. Procédé de préparation selon la revendication 10, caractérisé en ce que ladite solidification est réalisée par une opération physique telle qu'évaporation, dessiccation ou lyophilisation.

12. Procédé de préparation selon la revendication 10 ou la revendication 11, caractérisé en ce que lorsque la solidification est réalisée par lyophilisation, l'opération est conduite en deux temps :
(i) division de la pâte en quantités unitaires de forme et de volume prédéterminés, et
(ii) lyophilisation, étant entendu que les étapes (i) et (ii) peuvent être réalisées indifféremment dans n'importe quel ordre.

13. Procédé de préparation selon la revendication 10 ou la revendication 11, caractérisé en ce que lorsque la solidification est réalisée par évaporation, l'opération est conduite dans un mélangeur sécheur, sous pression réduite et exposition intermittente aux hyperfréquences.

14. Médicament, caractérisé en ce qu'il est constitué par une forme galénique selon l'une quelconque des revendications 1 à 9.

15. Vecteur immunologique, caractérisé en ce qu'il est constitué par une forme galénique selon l'une quelconque des revendications 1 à 9.

16. Produit diététique, caractérisé en ce qu'il comprend une forme galénique selon l'une quelconque des revendications 1 à 5.

17. Produit alimentaire, caractérisé en ce qu'il comprend une forme galénique selon l'une quelconque des revendications 1 à 5.

## Claims

1. Dosage form for orally administering chemical or medicinal substances, of the type comprising a solid central core containing one or more bioactive substances and an outer layer or coating, characterized in that:
- the porous water-soluble central core comprises:
· at least one substance chosen from the group which comprises the binders selected from polypeptides, high molecular weight polysaccharides, polymers capable of giving colloidal solutions and colloids, and diluents selected from polyols, metal oxides, carbonates, phosphates or micro-crystalline cellulose, the overall quantity of binder and diluent being between 50 and 98 % by weight relative to the said central core, and
· an effective quantity of at least one bioactive substance, and
- the palatable hydrophobic outer layer comprises:
· at least one lipid substance chosen from fatty alcohols, fatty acids, glycerol esters, hydrogenated oils, waxes, paraffin, lanolin, coconut oil and fatty acid salts;
· an agent for modulating the disintegration and the adhesion of the said outer layer, soluble or dispersible in the lipid mass, chosen from polymers selected from the group which comprises acrylic resins, polyvinyl acetates, polyalkylene resins, ethylene/vinyl acetate (EVA) copolymers, cross-linked polymers of starch, dextran, inulin or vinylpyrrolidone and cellulose derivatives selected from hydroxypropyl cellulose, hydroxypropyl methylcellulose, ethyl cellulose, carboxymethylcellulose; and
· natural or synthetic palatable substances.

2. Dosage form according to Claim 1, characterized in that the central core comprises, in addition, one or more additives chosen from taste modifying or masking substances, disintegration-modulating agents, absorption promoters, surface-active agents chosen from nonionic or cationic surfactants, colorants and preservatives.

3. Dosage form according to Claim 1 or Claim 2, characterized in that the outer layer comprises, in addition, inorganic substances chosen from the group which comprises inorganic oxides, phosphates, carbonates, clays and talc and/or a surfactant chosen from sorbitol esters, polyoxyethylene polysorbates, lecithins and sorbitan esters.

4. Dosage form according to any one of Claims 1 to 3, characterized in that the diluents and binders of the inner core are selected from gum arabic, xanthan gum, pectin, sorbitol, xylitol and mannitol type polyols and mixtures thereof.

5. Dosage form according to any one of Claims 1 to 4, characterized in that the lipid substance of the said outer layer is chosen from paraffin, coconut oil, palmitic acid and glycerol esters, and in that the agent for modulating the disintegration of the said outer layer is chosen from ethylene/vinyl acetate copolymers and cross-linked polysaccharides.

6. Dosage form according to any one of Claims 1 to 5, characterized in that the bioactive substances of the inner core are chosen from vaccines, cytokines, anti-cancer agents, growth factors, immunity adjuvants, transgenics-supporting factors, local attachment or anti-attachment factors.

7. Dosage form according to Claim 6, characterized in that the inner core comprises, as bioactive substance, a vaccine chosen from antiviral vaccines, antiparasitic vaccines, antibacterial vaccines and contraceptive vaccines.

8. Dosage form according to Claim 6 or Claim 7, characterized in that the inner core comprises, as bioactive substance, an anti-rabies vaccinal suspension of strain SAG-2.

9. Dosage form according to any one of Claims 1 to 8, characterized in that it has the following formula:
| - central core: | |
|---|---|
| diluents and/or binders | 50 to 98 % |
| bioactive substances | 0.25 to 50 % |
| other additives | 0 to 1.75 % |
| - outer layer: | |
|---|---|
| lipid substances | 40 to 93 % |
| polymers | 4 to 30 % |
| palatable substances and other additives | 3 to 30 % |

10. Method for preparing a dosage form according to any one of Claims 1 to 9, characterized in that the following operations are carried out:
a) preparation of a paste containing the various constituents of the central core, namely bioactive substances, diluents, binders and optionally one or more additives and a quantity of water suitable to adjust the homogeneity and the viscosity of the suspension obtained,
b) solidification of the product obtained in a),
c) coating of the products obtained in b), with a mixture in solution or in suspension, comprising at least one lipid substance, one agent for modulating the disintegration of the outer layer and one palatable substance.

11. Method of preparation according to Claim 10, characterized in that the said solidification is carried out by a physical operation such as evaporation, drying or freeze-drying.

12. Method of preparation according to Claim 10 or Claim 11, characterized in that when the solidification is carried out by freeze-drying, the operation is conducted in two stages:
(i) division of the paste into unit quantities of predetermined shape and volume, and
(ii) freeze-drying, it being understood that steps (i) and (ii) can be carried out in any order.

13. Method of preparation according to Claim 10 or Claim 11, characterized in that when the solidification is carried out by evaporation, the operation is conducted in a mixer-dryer, under reduced pressure and intermittent exposure to ultrahigh frequencies.

14. Medicinal product, characterized in that it consists of a dosage form according to any one of Claims 1 to 9.

15. Immunological vector, characterized in that it consists of a dosage form according to any one of Claims 1 to 9.

16. Dietetic product, characterized in that it comprises a dosage form according to any one of Claims 1 to 5.

17. Food product, characterized in that it comprises a dosage form according to any one of Claims 1 to 5.

## Patentansprüche

1. Galenische Form für die orale Verabreichung von chemischen oder medikamentösen Substanzen des Typs, umfassend einen festen mittleren Kern, enthaltend eine oder mehrere bioaktive Substanzen und eine äußere oder Umhüllungsschicht, dadurch **gekennzeichnet,** daß
- der mittlere wasserlösliche und poröse Kern umfaßt:
. mindestens eine Substanz, ausgewählt aus der Gruppe, umfassend Bindemittel, ausgewählt aus Polypeptiden, Polysacchariden mit erhöhtem Molekulargewicht, Polymeren mit der Fähigkeit, colloidale Lösungen ergeben zu können und Colloide und Verdünnungsmittel, ausgewählt aus Polyolen, Metalloxiden, Carbonaten, Phosphaten oder mikrokristalliner Cellulose, wobei die Gesamtmenge an Bindemittel und Verdünnungsmittel zwischen 50 und 98 Gew.-%, bezogen auf den mittleren Kern, liegt und
. eine wirksame Menge mindestens einer bioaktiven Substanz und
- die äußere Schicht mit hydrophober und appetitanregender Eigenschaft umfaßt:
. mindestens eine Lipidsubstanz, ausgewählt aus Fettalkoholen, Fettsäuren, Glycerinestern, hydrierten Ölen, Wachsen, Paraffin, Lanolin, Kopraöl und Salzen von Fettsäuren,
. ein Mittel zur Modulation des Zerfalls und der Adhäsion der äußeren Schicht, löslich oder dispergierbar in der Lipidmasse, ausgewählt aus Polymeren, ausgewählt aus der Gruppe, umfassend Acrylharze, Polyvinylacetate, Polyalkylenharze, Ethylen/Vinylacetatcopolymere (EVA), vernetzte Stärkepolymere, Dextran, Inulin oder Vinylpyrrolidon und Cellulosederivate, ausgewählt aus Hydroxypropylcellulose, Hydroxypropylmethylcellulose, Ethylcellulose, Carboxymethylcellulose und
. natürliche oder synthetische appetitanregende Substanzen.

2. Galenische Form nach Anspruch 1, dadurch **gekennzeichnet**, daß der mittlere Kern weiterhin ein oder mehrere Additiv(e), ausgewählt aus den Geschmack modifizierenden oder maskierenden Substanzen, Mitteln, die den Zerfall modulieren, Absorptionspromotoren, oberflächenaktiven Mitteln, ausgewählt aus nichtionischen oder kationischen grenzflächenaktiven Mitteln, Farbstoffen und Konservierungsstoffen, umfaßt.

3. Galenische Form nach Anspruch 1 oder 2, dadurch **gekennzeichnet,** daß die äußere Schicht weiterhin mineralische Substanzen, ausgewählt aus der Gruppe, umfassend Mineraloxide, Phosphate, Carbonate, Tone und Talk und/oder ein grenzflächenaktives Mittel, ausgewählt aus Sorbitestern, Polyoxiethylenpolysorbaten, Lezithinen und Sorbitanestern umfaßt.

4. Galenische Form nach einem der Ansprüche 1 bis 3, dadurch **gekennzeichnet,** daß die Verdünnungsmittel und Bindemittel des inneren Kerns aus Gummi arabicum, Xanthangummi, Pektin, Polyolen vom Sorbit-, Xylit-Manittyp und ihren Gemischen ausgewählt sind.

5. Galenische Form nach einem der Ansprüche 1 bis 4, dadurch **gekennzeichnet,** daß die Lipidsubstanz der äußeren Schicht aus Paraffn, Kopraöl, Palmitinsäure und Glycerinestern ausgewählt ist, und daß das Mittel zur Modulation des Zerfalls der äußeren Schicht aus Ethylen/Vinylacetatcopolymeren und vernetzten Polysacchariden ausgewählt ist.

6. Galenische Form nach einem der Ansprüche 1 bis 5, dadurch **gekennzeichnet,** daß die bioaktiven Substanzen des inneren Kerns aus Impfstoffen, Zytokinen, Antikrebsmitteln, Wachstumsfaktoren, Immunadjuvantien, transgenen Unterstützungsfaktoren, Faktoren für oder gegen die lokale Anheftung ausgewählt sind.

7. Galenische Form nach Anspruch 6, dadurch **gekennzeichnet,** daß der innere Kern als bioaktive Substanz einen Impfstoff, ausgewählt aus antiviralen Impfstoffen, antiparasitären Impfstoffen, antibakteriellen Impfstoffen, kontrazeptiven Impfstoffen umfaßt.

8. Galenische Form nach Anspruch 6 oder 7, dadurch **gekennzeichnet,** daß der innere Kern als bioaktive Substanz eine Impfsuspension gegen Tollwut des Stammes SAG-2 umfaßt.

9. Galenische Form nach einem der Ansprüche 1 bis 8, dadurch **gekennzeichnet,** daß sie in folgender Formulierung vorliegt:
| - mittlerer Kern | |
|---|---|
| Verdünnungsmittel und/oder Bindemittel | 50 bis 98% |
| bioaktive Substanzen | 0,25 bis 50% |
| weitere Additive | 0 bis 1,75% |
| - äußere Schicht | |
|---|---|
| Lipidsubstanzen | 40 bis 93% |
| Polymere | 4 bis 30% |
| appetitanregende Substanzen und andere Additive | 3 bis 30% |

10. Verfahren zur Herstellung einer galenischen Form nach einem der Ansprüche 1 bis 9, dadurch **gekennzeichnet,** daß man die folgenden Arbeitsschritte durchführt:
(a) Herstellung einer Paste, enthaltend die verschiedenen Bestandteile des mittleren Kerns, d.h. bioaktive Substanzen, Verdünnungsmittel, Bindemittel und gegebenenfalls ein oder mehrere Additiv(e) und eine geeignete Menge Wasser zur Regulierung der Homogenität und der Viskosität der so erhaltenen Suspension;
(b) Verfestigung des in (a) erhaltenen Produkts;
(c) Umhüllung der in (b) erhaltenen Produkte mit einem Gemisch in Lösung oder in Suspension, umfassend mindestens eine Lipidsubstanz, ein Mittel zur Modulation des Zerfalls der äußeren Schicht und eine appetitanregende Substanz.

11. Verfahren zur Herstellung nach Anspruch 10, dadurch **gekennzeichnet,** daß die Verfestigung durch einen physikalischen Arbeitsschritt wie Eindampfen, Austrocknen oder Gefriertrocknen durchgeführt wird.

12. Verfahren zur Herstellung nach Anspruch 10 oder 11, dadurch **gekennzeichnet,** daß, wenn die Verfestigung durch Gefriertrocknung durchgeführt wird, die Arbeit in zwei Zeitstufen durchgeführt wird:
(i) Verteilen der Paste in Einheitsmengen von vorbestimmter Form und vorbestimmtem Volumen und
(ii) Lyophilisation mit der Maßgabe, daß die Stufen (i) und (ii) in jeder beliebigen Reihenfolge ohne Unterschied durchgeführt werden können.

13. Verfahren zur Herstellung nach Anspruch 10 oder 11, dadurch **gekennzeichnet,** daß, wenn die Verfestigung durch Eindampfen durchgeführt wird, die Arbeit in einen Trockenmischer unter vermindertem Druck und intermittierender Exposition gegenüber Hyperfrequenzen durchgeführt wird.

14. Medikament, dadurch **gekennzeichnet,** daß es aus einer galenischen Form nach einem der Ansprüche 1 bis 9 besteht.

15. Immunologischer Vektor, dadurch **gekennzeichnet,** daß er aus einer galenischen Form nach einem der Ansprüche 1 bis 9 besteht.

16. Diätetisches Produkt, dadurch **gekennzeichnet,** daß es eine galenische Form nach einem der Ansprüche 1 bis 5 umfaßt.

17. Lebensmitttelprodukt, dadurch **gekennzeichnet,** daß es eine galenische Form nach einem der Ansprüche 1 bis 5 umfaßt.
